# EUROPEAN PATENT APPLICATION

(11) **EP 1 759 715 A1**
(43) Date of publication of application: **07.03.2007**
(21) Application number: 06731277.7
(22) Date of filing: 06.04.2006
(51) Int. Cl.: A61L 2/20, B01D 53/26

(54) **STERILIZATION SYSTEM**

(30) Priority: 08.04.2005 JP 2005112584
(71) Applicant: DAIKIN INDUSTRIES, LTD., Osaka-shi, Osaka 530-8323 (JP)
(72) Inventor: HIRANO, Yoshikazu c/o DAIKIN APPLIED SYSTEMS, Tokyo 1080023 (JP); MIYAZAWA, Osamu c/o DAIKIN APPLIED SYSTEMS, Tokyo 1080023 (JP)
(74) Representative: Goddar, Heinz J.
(86) International application number: PCT/JP2006/007329
(87) International publication number: WO 2006/109670

(57) **Abstract**

In a sterilization system including a hydrogen peroxide generator (51) for generating hydrogen peroxide and an introduction passage (20) for introducing hydrogen peroxide generated in the hydrogen peroxide generator (51) into a treatment space (2) to be sterilized, a dehumidifier (52) for dehumidifying air is provided. The introduction passage (20) comprises an air introduction passage (21) for introducing the air having passed through the dehumidifier (52) into the treatment space (2) and a hydrogen peroxide introduction passage (22) connected to the hydrogen peroxide generator (51) and the air introduction passage (21) at a position between the dehumidifier (52) and the treatment space (2). Thus, in the sterilization system for treating the interior of the treatment space (2) with hydrogen peroxide, the hydrogen peroxide concentration in the treatment space (2) is made uniform whereby the treatment space (2) can provide a uniform sterilization capacity as a whole while the system is prevented from cost rise.

## Description

### Technical Field

This invention relates to sterilization systems for subjecting the interior of a treatment space to a sterilization process with hydrogen peroxide.

### Background Art

Conventional sterilization systems of such kind include a system in which a treatment device for drugs is put as a sterilization object in an isolator defining a closed space and sterilized in the isolator (see, for example, Patent Document 1).

In the sterilization system of Patent Document 1, the isolator is connected with an outdoor air supply circuit and a hydrogen peroxide supply circuit. The outdoor air supply circuit is configured to introduce outdoor air into the isolator with an air supply fan. Further, the hydrogen peroxide supply circuit is provided with a hydrogen peroxide generator for generating hydrogen peroxide as a sterilant, an intake port of the hydrogen peroxide supply circuit is connected to an exhaust duct disposed at a lower part of the isolator and a gas supply port of the circuit is connected to an outdoor air introduction chamber disposed at an upper part of the isolator.

Furthermore, after the introduction of outdoor air through the outdoor air supply circuit into the isolator, the sterilization system supplies, as a sterilant, hydrogen peroxide generated in the hydrogen peroxide generator into the isolator. Thereafter, the sterilization system circulates the gas containing hydrogen peroxide in the isolator by returning the gas from the exhaust duct of the isolator through the hydrogen peroxide supply circuit to the outdoor air introduction chamber of the isolator.
Patent Document 1: Published Japanese Patent Application No. 2001-000514

### Disclosure of the Invention

### Problems to Be Solved by the Invention

However, since the above conventional sterilization system introduces hydrogen peroxide into the treatment space in the isolator after the supply of outdoor air into the treatment space, this causes a problem that the hydrogen peroxide concentration in the treatment space is likely to be lacking in uniformity. Therefore, the treatment space includes an area providing a sufficient sterilization capacity and an area lacking in sterilization capacity (a so-called dead spot), so that the treatment space cannot provide a uniform sterilization capacity as a whole.

Alternatively, in order to make the hydrogen peroxide concentration in the treatment space uniform, a method can be devised for stirring the interior air with a blower. This method, however, involves cost rise of the system.

The present invention has been made in view of the above points and, therefore, its object is to provide a sterilization system for treating the interior of a treatment space with hydrogen peroxide wherein the hydrogen peroxide concentration in the treatment space is made uniform so that the treatment space can provide a uniform and sufficient sterilization capacity as a whole while the system is prevented from cost rise.

### Means to Solve the Problems

A first solution of the present invention is directed to a sterilization system comprising a hydrogen peroxide generator **(51)** for generating hydrogen peroxide and an introduction passage **(20)** for introducing hydrogen peroxide generated in the hydrogen peroxide generator **(51)** into a treatment space **(2)** to be sterilized.

Further, the sterilization system is characterized by comprising a dehumidifier **(52)** for dehumidifying air and characterized in that the introduction passage **(20)** comprises: an air introduction passage **(21)** for introducing the air having passed through the dehumidifier **(52)** into the treatment space **(2);** and a hydrogen peroxide introduction passage **(22)** connecting the hydrogen peroxide generator **(51)** to the air introduction passage **(21)** at a position between the dehumidifier **(52)** and the treatment space **(2).**

In the first solution, when air passes through the dehumidifier **(52),** moisture in the air is removed by the dehumidifier **(52)** to dehumidify the air. In the meantime, the hydrogen peroxide generator **(51)** generates hydrogen peroxide. The generated hydrogen peroxide flows through the hydrogen peroxide introduction passage **(22)** and then merges into the dehumidified air in the air introduction passage **(21).** At the time of merging, since the air has a low humidity, hydrogen peroxide instantly evaporates to uniformly disperse in the air. Thereafter, the air containing hydrogen peroxide is introduced through the introduction passage **(20)** into the treatment space **(2)** and sterilize the treatment space **(2).**

A second solution of the present invention is directed to the first solution and
characterized by further comprising a circulation passage **(40),** connected to an exhaust port **(2a)** provided at the treatment space **(2)** and an air inlet side of the dehumidifier **(52),** for introducing gas in the treatment space **(2)** into the dehumidifier **(52).**

In the second solution, since the air in the treatment space **(2)** is circulated through the circulation passage **(40)** in the direction from outlet side to inlet side of the treatment space **(2),** air can be used without wasting it, thereby providing an efficient operation.

A third solution of the present invention is directed to the first solution and
characterized in that the dehumidifier **(52)** comprises: an adsorption rotor **(53)** that is disposed to span a first passage **(P1)** allowing a first air to flow and a second passage **(P2)** allowing a second air to flow, rotatable about a point between the first passage **(P1)** and the second passage **(P2)** and capable of adsorbing moisture in the first air; and a heater **(58)** disposed in the second passage **(P2)** upstream of the adsorption rotor **(53).**

According to the third solution, in the first passage **(P1),** when the first air passes through the adsorption rotor **(53),** moisture in the first air is adsorbed on the adsorbent in the adsorption rotor **(53).** In other words, the first air is dehumidified. In the second passage **(P2),** when the second air heated by the heater **(58)** passes through the adsorption rotor **(53),** moisture held by the adsorbent in the adsorption rotor **(53)** is desorbed to regenerate the adsorption rotor **(53).** The adsorption rotor **(53)** is configured to be rotatable about a point between the first passage **(P1)** and the second passage **(P2).** Thus, when the side of the adsorption rotor **(53)** located across the first passage **(P1)** and having adsorbed moisture moves to the second passage **(P2)** side by the rotation of the adsorption rotor **(53),** it in turn releases moisture to the second air and is thereby regenerated. On the other hand, when the side of the adsorption rotor **(53)** located across the second passage **(P2)** and regenerated therein moves to the first passage **(P1)** side by the rotation of the adsorption rotor **(53),** it in turn adsorbs moisture in the first air.

A fourth solution of the present invention is directed to the third solution and
characterized in that the dehumidifier **(52)** further comprises: a temperature/humidity sensor **(62)** disposed in the first passage **(P1)** downstream of the adsorption rotor **(53);** and a regulator (64) for regulating the heating capacity of the heater **(58)** based on the detected value of the temperature/humidity sensor **(62).**

In the fourth solution, the temperature and humidity of the processed first air having passed through the adsorption rotor **(53)** in the first passage **(P1)** is detected to operate its absolute humidity and the amount of heat for heating the second air flowing through the second passage **(P2)** is controlled to an appropriate value based on the absolute humidity. Thus, the humidity of the first air having passed through the adsorption rotor **(53)** can be kept constant (or, alternatively, the humidity of the first air passed through the adsorption rotor **(53)** can be controlled to be constant also if the dew point of the processed first air is detected instead of the temperature and humidity thereof). In other words, the humidity of the first air flowing through the side of the adsorption rotor **(53)** located across the first passage **(P1)** can be controlled by regulating the amount of regeneration in the side of the adsorption rotor **(53)** located across the second passage **(P2).** Specifically, when the first air has a higher humidity than a set value, the amount of regeneration in the second passage **(P2)** is set at a larger value in order to increase the amount of adsorption in the first passage **(P1).** When the first air has a lower humidity than the set value, the amount of regeneration in the second passage **(P2)** is set at a smaller value in order to decrease the amount of adsorption in the first passage **(P1).**

A fifth solution of the present invention is directed to the fourth solution and
characterized in that the dehumidifier **(52)** further comprises coolers **(55, 57)** in the first passage **(P1),** one disposed upstream of the adsorption rotor **(53)** and one disposed downstream of the adsorption rotor **(53)** between the adsorption rotor (53) and the temperature/humidity sensor **(62).**

In the fifth invention, the air cooled by the coolers **(55, 57)** and dehumidified by the adsorption rotor **(53)** are mixed with hydrogen peroxide and then supplied to the treatment space **(2).** Particularly, since the first air is cooled upstream of the adsorption rotor **(53),** this enhances the adsorption capacity of the adsorption rotor **(53)** and supplies a lower-humidity air to the treatment space **(2).**

A sixth solution of the present invention is directed to the first solution and
characterized in that elements of the dehumidifier **(52)** are each made of material corrosion-resistant to hydrogen peroxide.

In the sixth solution, even if hydrogen peroxide flows through the elements of the dehumidifier **(52),** the elements are not corroded by hydrogen peroxide.

### Effects of the Invention

In the present invention, the introduction passage **(20)** comprises the air introduction passage **(21)** for introducing air having passed through the dehumidifier **(52)** into the treatment space **(2)** and the hydrogen peroxide introduction passage **(22)** connecting the hydrogen peroxide generator **(51)** to the air introduction passage **(21)** at a position between the dehumidifier **(52)** and the treatment space **(2).** Therefore, when hydrogen peroxide generated in the hydrogen peroxide generator **(51)** merges into dehumidified air in the air introduction passage **(21)** after flowing through the hydrogen peroxide introduction passage **(22),** hydrogen peroxide instantly evaporates and disperses uniformly into the dehumidified air. Then, the air containing hydrogen peroxide is introduced through the introduction passage **(20)** into the treatment space **(2).** Therefore, the hydrogen peroxide concentration in the treatment space **(2)** can be uniform, thereby preventing the occurrence of a dead spot. Further, there is no need to provide a fan for stirring the interior of the treatment space **(2),** which prevents cost rise of the system.

According to the second solution, since the circulation passage **(40)** is provided which is connected to the exhaust port **(2a)** provided at the treatment space **(2)** and the air inlet side of the dehumidifier **(52)** to introduce gas in the treatment space **(2)** into the dehumidifier **(52),** the air in the treatment space **(2)** can be circulated through the circulation passage **(40)** in the direction from outlet side to inlet side of the treatment space **(2).** This provides an efficient operation using air without wasting it.

According to the third solution, since the adsorption rotor **(53)** rotatable around a point between the first passage **(P1)** and the second passage **(P2)** is used and the heater **(58)** is disposed in the second passage **(P2)** upstream of the adsorption rotor **(53),** an operation can be continuously carried out to dehumidify the first air in the side of the adsorption rotor **(53)** located across the first passage **(P1)** and concurrently regenerate the side of the adsorption rotor **(53)** located across the second passage **(P2).** Hence, the air supplied to the treatment space **(2)** (the first air) is always a low-humidity air and hydrogen peroxide is mixed into the low-humidity air. This enables the hydrogen peroxide concentration in the treatment space **(2)** to be always kept uniform.

According to the fourth solution, since the dehumidifier **(52)** is provided with the temperature/humidity sensor **(62)** disposed in the first passage **(P1)** downstream of the adsorption rotor **(53)** and the regulator **(64)** for regulating the heating capacity of the heater **(58)** based on the detected value of the temperature/humidity sensor **(62),** the temperature and humidity of the processed first air having passed through the adsorption rotor **(53)** in the first passage **(P1)** can be detected to operate its absolute humidity and the amount of heat for heating the second air flowing through the second passage **(P2)** can be controlled to an appropriate value based on the obtained absolute humidity. In other words, when the first air has a higher humidity than the set value, the amount of regeneration in the second passage **(P2)** can be set at a larger value in order to increase the amount of adsorption in the first passage **(P1).** When the first air has a lower humidity than the set value, the amount of regeneration in the second passage **(P2)** can be set at a smaller value in order to decrease the amount of adsorption in the first passage **(P1).** This provides a control to always keep the humidity of the first air constant.

It is generally known that, in sterilization systems of this kind, a higher sterilization effect can be obtained at a lower humidity in the treatment space **(2).** However, even if in conventional sterilization systems a low-humidity air is used as an air supplied into the isolator, the supply of hydrogen peroxide after the supply of low-humidity air into the space of the isolator causes humidity rise in the space, thereby deteriorating the sterilization effect. In contrast, since the present invention can provide a control to always keep the humidity of the first air constant, the deterioration of the sterilization effect can be also prevented.

According to the fifth solution, since the coolers **(55, 57)** are disposed in the first passage **(P1),** one upstream of the adsorption rotor **(53)** and one downstream of the adsorption rotor **(53)** between the adsorption rotor **(53)** and the temperature/humidity sensor **(62),** the first air is cooled by the coolers **(55, 57)** and dehumidified by the adsorption rotor **(53),** followed by mixture with hydrogen peroxide and supply to the treatment space **(2).** Particularly, since the first air is cooled upstream of the adsorption rotor **(53),** this enhances the adsorption capacity of the adsorption rotor **(53)** and supplies a lower-humidity air to the treatment space **(2).**

According to the sixth solution, since the elements of the dehumidifier **(52)** are each made of material corrosion-resistant to hydrogen peroxide, they are not corroded by hydrogen peroxide even if hydrogen peroxide flows through them. Therefore, the dehumidifier **(52)** is not immediately damaged, which enables to design a good working sterilization system.

### Brief Description of Drawings

[Fig. **1]** Figure **1** is block diagram of a sterilization system according to an embodiment of the present invention.
[Fig. **2]** Figure **2** is a schematic block diagram of a sterilizer.
[Fig. **3]** Figure **3** is a block diagram of a dehumidifier.
[Fig. **4]** Figure **4** is a detailed block diagram showing essential parts of the dehumidifier.

### Explanation of Reference Characters

- **1**: sterilization system
- **2**: treatment space
- **2a**: exhaust port
- **20**: introduction passage
- **21**: air introduction passage
- **22**: hydrogen peroxide introduction passage
- **40**: circulation passage
- **51**: hydrogen peroxide generator
- **52**: dehumidifier
- **53**: adsorption rotor
- **55**: pre-cooler (cooler)
- **57**: after-cooler (cooler)
- **58**: heater
- **62**: temperature/humidity sensor
- **64**: regulator
- **P1**: first passage
- **P2**: second passage

### Best Mode for Carrying Out the Invention

An embodiment of the present invention will de described below in detail with reference to the drawings.

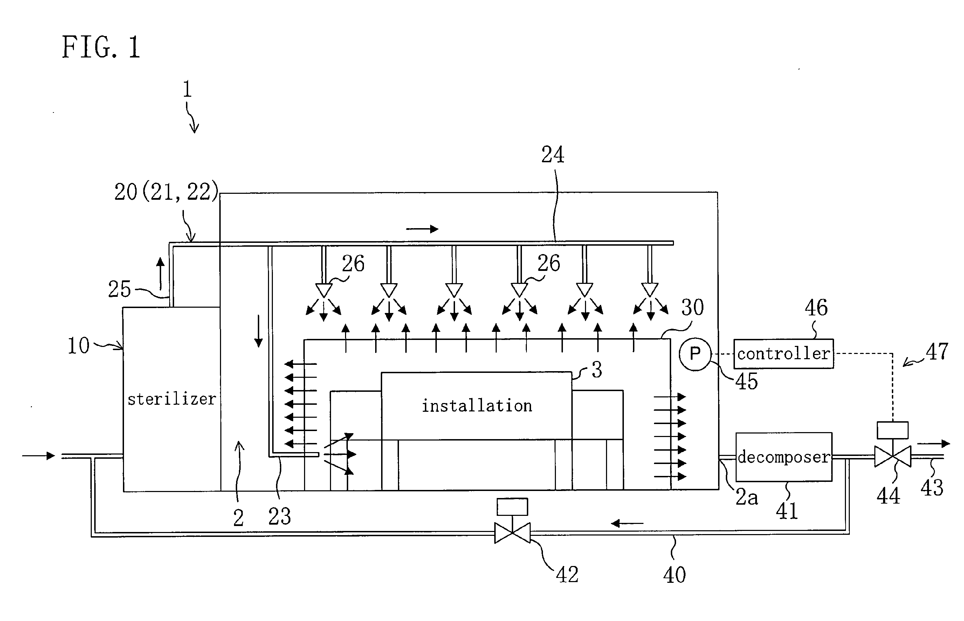

Figure **1** is a block diagram of a sterilization system **(1)** according to this embodiment. The sterilization system **(1)** includes a sterilizer **(10)** for generating hydrogen peroxide as an sterilant and an introduction passage **(20)** for introducing a treatment gas containing hydrogen peroxide generated in the sterilizer **(10)** into a treatment space **(2).** As described later, the introduction passage **(20)** includes an air introduction passage **(21)** and a hydrogen peroxide introduction passage **(22).** The treatment space **(2)** is a room space to be sterilized in which an installation **(3),** such as a drug production line, is installed as a sterilization object. The sterilizer **(10)** is placed outside the treatment space **(2).** The interior of the treatment space **(2)** is provided with a concentration sensor (not shown) for detecting the concentration of hydrogen peroxide in the space **(2).**

The sterilization system **(1)** is provided with a gas-permeable cover member **(30)** covering the installation **(3)** in the treatment space **(2).** The cover member **(30)** is made of porous material and can be fabricated, for example, using non-woven fabric.

The introduction passage **(20)** includes a first introduction passage **(23)** for releasing hydrogen peroxide generated in the sterilizer **(10)** therethrough to the interior of the cover member **(30)** thereby allowing the generated hydrogen peroxide to be supplied through gas-permeable pores in the cover member **(30)** into the treatment space **(2),** and a second introduction passage **(24)** for introducing hydrogen peroxide generated in the sterilizer **(10)** directly into the treatment space **(2).** The first introduction passage **(23)** and the second introduction passage **(24)** are provided by branching from a main pipe **(25)** connected to the sterilizer **(10).**

The drug production line **(3)** is installed in a lower area of the treatment space **(2)** and the cover member **(30)** is configured to cover the whole drug production line **(3)** from above. Further, the first introduction passage **(23)** is connected to a lower part of the cover member **(30)** and the second introduction passage **(24)** is placed in the treatment space **(2)** above the cover member **(30).** Though not shown, the second introduction passage (24) is provided in the form of a plurality of rows to cover the whole treatment space **(2).** Further, each row of the second introduction passage **(24)** is provided with a plurality of blow-off nozzles **(26)** for blowing off hydrogen peroxide downward and obliquely downward therefrom.

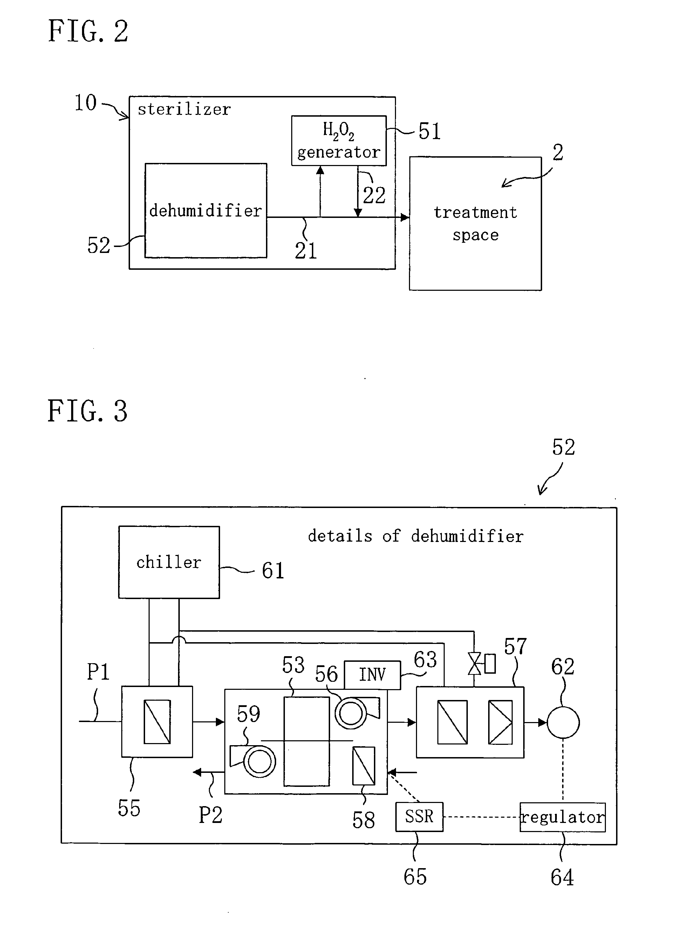

The treatment space **(2)** is provided with an exhaust port **(2a).** The exhaust port **(2a)** and the sterilizer **(10)** are connected to a circulation passage **(40)** for returning gas in the treatment space **(2)** to the sterilizer **(10)** and supplying the gas from the sterilizer **(10)** into the treatment space **(2)** again. To be more specific, the circulation passage **(40)** is connected to the exhaust port **(2a)** and the air inlet side of a dehumidifier **(52)** described later (see Figures **2** to **4),** and configured to introduce gas in the treatment space **(2)** into a first passage **(P1)** (see the description below) in the dehumidifier **(52).** The circulation passage **(40)** is provided with a decomposer **(41)** for decomposing hydrogen peroxide and a circulation flow control valve **(42)** for controlling the flow volume of gas in the circulation passage **(40).**

The circulation passage **(40)** is connected to an exhaust passage **(43)** branched at a position on the circulation passage **(40)** downstream of the decomposer **(41).** The exhaust passage **(43)** is provided with an exhaust flow control valve **(44)** for controlling the flow volume of gas in the exhaust passage **(43)** (the flow volume of exhaust gas from the treatment space **(2)).** The gas exhausted from the treatment space **(2)** is a gas from which hydrogen peroxide was decomposed off during passage through the decomposer **(41).**

The interior of the treatment space **(2)** is provided with a pressure sensor **(45)** for detecting the pressure in the treatment space **(2).** Connected between the pressure sensor **(45)** and the exhaust flow control valve **(44)** is a controller **(46)** for controlling the opening of the exhaust flow control valve **(44)** based on the detected value of the pressure sensor **(45).** Further, the exhaust flow control valve **(44),** the pressure sensor **(45)** and the controller **(46)** constitute a pressure control mechanism **(47)** for controlling the pressure in the treatment space **(2).** The pressure control mechanism **(47)** is configured to hold the pressure in the treatment space **(2)** at a higher value than the pressure in the exterior space.

Next, a description is given of a specific structure of the sterilizer **(10)** with reference to Figures **2** to **4.** Figure **2** is a schematic block diagram of the sterilizer **(10).** As shown in Figure **2,** the sterilizer **(10)** includes a hydrogen peroxide generator **(51)** for generating hydrogen peroxide serving as a sterilant and the above-mentioned dehumidifier **(52)** for dehumidifying air.

As described above, the introduction passage **(20)** includes the air introduction passage **(21)** and the hydrogen peroxide introduction passage **(22).** The air introduction passage **(21)** is configured to introduce air having passed through the dehumidifier **(52)** into the treatment space **(2),** while the hydrogen peroxide introduction passage **(22)** is provided to introduce hydrogen peroxide generated in the hydrogen peroxide generator **(51)** into the treatment space **(2)** to be sterilized. Further, the hydrogen peroxide generator **(51)** is connected to the air introduction passage **(21)** between the dehumidifier **(52)** and the treatment space **(2).** Thus, a gas mixture of air dehumidified during passage through the dehumidifier **(52)** and hydrogen peroxide is introduced into the treatment space **(2).**

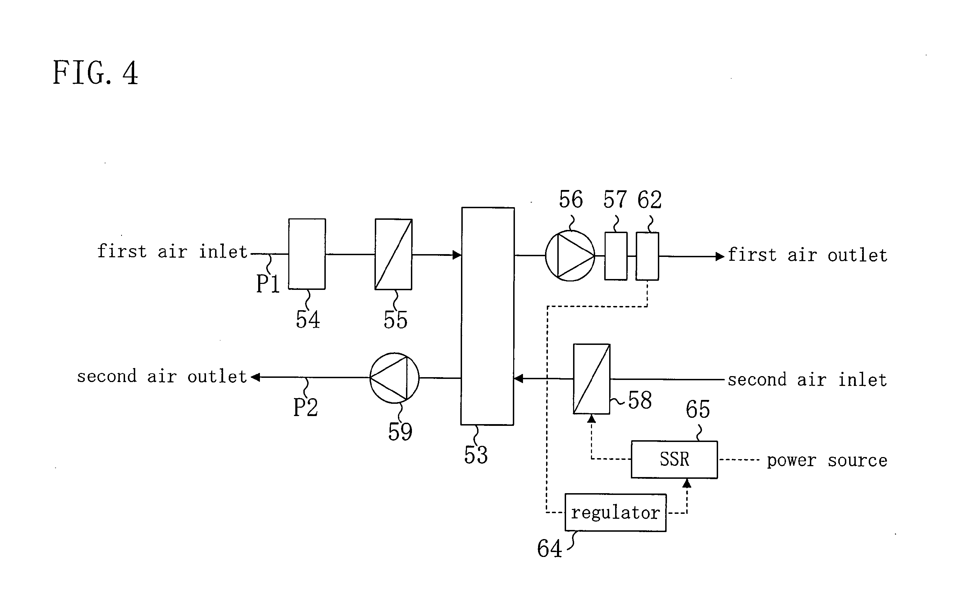

Figure **3** is a block diagram of the dehumidifier **(52)** and Figure **4** is a detailed block diagram showing essential parts of the dehumidifier **(52).** The dehumidifier **(52)** is a dehumidifier **(52)** of a type using an adsorption rotor **(53)** in which a honeycombed disk member having axial gas-permeability is used as a base material and an adsorbent is carried on the surface of the disk member. The adsorption rotor **(53)** is capable of moisture adsorption and desorption and is disposed to span the first passage **(P1)** for adsorption allowing a first air to flow and the second passage **(P2)** for desorption (regeneration) allowing a second air to flow. In the adsorption side of the adsorption rotor **(53),** moisture in the first air is adsorbed on the adsorbent in the adsorption rotor **(53)** to dehumidify the first air. In the desorption side of the adsorption rotor **(53),** moisture adsorbed on the adsorbent in the adsorption rotor **(53)** is released to the second air to regenerate the adsorption rotor **(53).**

In addition, the adsorption rotor **(53)** is configured to continuously or intermittently rotate about a point between the first passage **(P1)** and the second passage **(P2).** Thus, when the side of the adsorption rotor **(53)** located across the first passage **(P1)** and having adsorbed moisture therein moves to the second passage **(P2)** side, it in turn releases moisture to the second air and is thereby regenerated. Next time the side of the adsorption rotor **(53)** located across the second passage **(P2)** and regenerated therein moves to the first passage **(P1)** side, it in turn adsorbs moisture in the first air.

The circulation passage **(40)** is connected to the air inlet side of the first passage **(P1).** In the first passage **(P1)** upstream of the adsorption rotor **(53),** a pre-filter **(54)** for removing dusts in the first air and a pre-cooler **(55)** (cooler) for cooling the first air are disposed in order of the flow direction of the first air. In the first passage **(P1)** downstream of the adsorption rotor **(53),** a processing fan **(56)** for generating an air flow of the first air, an after-cooler **(57)** (cooler) for cooling the first air and a temperature/humidity sensor **(62)** for detecting the temperature and humidity of the processed first air are disposed in order of the flow direction of the first air.

The pre-cooler **(55)** and the after-cooler (57) are both cross fin type fin-and-tube heat exchangers for cooling air with cool water flowing therethrough. Though not shown in detail, the pre-cooler **(55)** and the after-cooler **(57)** are both connected to a chiller **(61)** including a refrigerant circuit operating in a vapor compression refrigeration cycle and configured so that cool water cooled by refrigerant in the refrigerant circuit flows through the tubes.

In the second passage **(P2)** upstream of the adsorption rotor **(53),** a heater **(58)** for heating the second air is disposed. Further, in the second passage **(P2)** downstream of the adsorption rotor **(53),** a regeneration fan **(59)** for generating an air flow of the second air is disposed. The processing fan **(56)** and the regeneration fan **(59)** each allow its motor to be controlled in rotational speed by an inverter **(63)** and thereby can be controlled in its amount of air flow.

The dehumidifier **(52)** is provided with a regulator **(64)** for regulating the heating capacity of the heater **(58)** based on the detected value of the temperature/humidity sensor **(62)** and a voltage converter **(65)** connected to a power source (not shown). Further, the temperature/humidity sensor **(62)** is connected to the regulator **(64)** and the regulator **(64)** is connected through the voltage converter **(65)** to the heater **(58).** According to this configuration, the temperature and humidity of the processed air in the first passage **(P1)** can be detected to operate its absolute humidity and the amount of heat applied by the heater **(58)** can be controlled based on the obtained absolute humidity, thereby keeping the humidity of the first air having passed through the adsorption rotor **(53)** constant.

In the dehumidifier (52) of this embodiment, the gas returning from the treatment space **(2)** upon deactivation of the decomposer **(41)** contains hydrogen peroxide. Therefore, the elements of the dehumidifier **(52)** including the adsorption rotor **(53),** the pre-filter **(54),** pre-cooler **(55),** the processing fan **(56),** the after-cooler **(57)** and the temperature/humidity sensor **(62)** are each made of material having corrosion resistance to hydrogen peroxide. For example, aluminium can be used as a material of such kind. However, the material used is not limited to aluminium and in turn any materials having the above corrosion resistance can be appropriately used.

### - Operational Behavior -

Next, a description is given of the operational behavior of the sterilization system **(1)** according to this embodiment.

First, the behavior during sterilization in Figure **1** is described. When the sterilizer **(10)** is turned on, hydrogen peroxide generated in the sterilizer **(10)** flows through the introduction passage **(20)** together with air and is distributed into the first introduction passage **(23)** and the second introduction passage **(24).**

Hydrogen peroxide in the first introduction passage **(23)** is introduced into the cover member **(30)** covering the drug production line **(3)** and prevails uniformly even to sites likely to become lightened in hydrogen peroxide concentration, such as surroundings of the foots of various devices on the drug production line **(3).** Since the cover member **(30)** is porous, hydrogen peroxide blows off from the cover member **(30)** upon filling up the interior of the cover member **(30).**

Hydrogen peroxide in the second introduction passage **(24)** is directly blown off into the treatment space **(2)** through a plurality of blow-off nozzles **(26)** provided in the second introduction passage **(24).** The pressure in the treatment space **(2)** is set higher than that in the exterior space by controlling the opening of the exhaust flow control valve **(44)** disposed in the exhaust passage **(43).** Thus, microbes can be prevented from invading from the exterior.

When it is detected from the detected value of an unshown concentration sensor that the concentration of hydrogen peroxide in the treatment space **(2)** reaches a predetermined value, the opening of the circulation flow control valve **(42)** disposed in the circulation passage **(40)** is controlled. Thus, an operation is carried out to pass the gas in the treatment space **(2)** through the decomposer **(41),** the circulation passage **(40),** the sterilizer **(10)** and the introduction passage **(20)** in this order and return it into the treatment space **(2)** again. In other words, the gas circulates through the closed circuit.

After decomposed by the decomposer **(41),** the gas containing hydrogen peroxide can be partly exhausted through the exhaust flow control valve **(44)** disposed in the exhaust passage **(43).** During the exhaust, as described above, the pressure in the room space is set higher than that in the exterior space by controlling the opening of the exhaust flow control valve (44).

Next, a description is given of a specific air treatment in the sterilizer **(10).**

In relation to the first passage **(P1)** side of the adsorption rotor **(53),** the air having returned from the treatment space **(2)** (the first air) passes through the pre-filter **(54)** so that dusts contained in the first air are removed by the pre-filter **(54),** and the first air is then cooled by the pre-cooler **(55).** During passage of the first air through the adsorption rotor **(53),** moisture in the first air is adsorbed on the adsorbent so that the first air is dehumidified. The dehumidified first air passes through the processing fan **(56)** and is then cooled by the after-cooler **(57).** Then, the first air is detected in terms of temperature and humidity by the temperature/humidity sensor **(62)** and then flows through the air introduction passage **(21)** toward the treatment space **(2).**

In the meantime, hydrogen peroxide generates in the hydrogen peroxide generator **(51).** The generated hydrogen peroxide passes through the hydrogen peroxide introduction passage **(22)** and then merges into the first air. Since the first air is a low-humidity air, the mixture thereof with hydrogen peroxide causes hydrogen peroxide to instantly evaporate and disperse uniformly into the first air.

The first air thus containing hydrogen peroxide in uniformly dispersed form is supplied through the first introduction passage **(23)** to the interior of the cover member **(30)** and concurrently supplied through the second introduction passage **(24)** into the treatment space **(2)** outside the cover member **(30).** Thus, the treatment space **(2)** is put under a condition that hydrogen peroxide is uniformly dispersed throughout the space. Particularly, in the interior of the cover member **(30),** hydrogen peroxide uniformly prevails even to so-called dead spots likely to be produced around the drug production line (3) (i.e., sites where the hydrogen peroxide concentration would normally become lower than in their surroundings).

In relation to the second passage **(P2)** side of the adsorption rotor **(53),** during flowing of the second air for regeneration through the heater **(58),** the amount of heat applied by the heater **(58)** is controlled according to the detected value of the temperature/humidity sensor **(62).** Thus, the amount of regeneration in the second passage **(P2)** side of the adsorption rotor **(53)** is controlled. Specifically, when the first air has a higher humidity than the set value, the amount of regeneration in the second passage **(P2)** is set at a larger value in order to increase the amount of adsorption in the first passage **(P1).** When the first air has a lower humidity than the set value, the amount of regeneration in the second passage **(P2)** is set at a smaller value in order to decrease the amount of adsorption in the first passage **(P1).** This provides a control to always keep the humidity of the first air constant. Note that the regenerated air having passed through the adsorption rotor (53) in the second passage (P2) is exhausted through the regeneration fan **(59)** to the exterior.

### - Effects of Embodiment -

In this embodiment, the introduction passage **(20)** comprises the air introduction passage **(21)** for introducing air having passed through the dehumidifier **(52)** into the treatment space **(2)** and the hydrogen peroxide introduction passage **(22)** connecting the hydrogen peroxide generator **(51)** to the air introduction passage **(21)** at the position between the dehumidifier **(52)** and the treatment space **(2).** Therefore, when hydrogen peroxide generated in the hydrogen peroxide generator **(51)** merges into dehumidified air in the air introduction passage **(21)** after flowing through the hydrogen peroxide introduction passage **(22),** hydrogen peroxide instantly evaporates and disperses uniformly into dehumidified air. Then, the air containing hydrogen peroxide is introduced through the introduction passage **(20)** into the treatment space **(2).** Therefore, the hydrogen peroxide concentration in the treatment space **(2)** can be uniform, thereby effectively preventing the occurrence of a dead spot. Further, there is no need to provide a fan for stirring the interior of the treatment space **(2),** which prevents cost rise of the system.

Furthermore, since the circulation passage **(40)** is connected to the exhaust port **(2a)** provided at the treatment space **(2)** and the air inlet side of the dehumidifier **(52)** to introduce gas in the treatment space **(2)** into the dehumidifier **(52),** the air in the treatment space **(2)** can be circulated through the circulation passage **(40)** in the direction from outlet side to inlet side of the treatment space **(2).** This provides an efficient operation using air without wasting it. Particularly, since air is dehumidified while being circulated, this provides a high sterilization effect and facilitates humidity control.

Furthermore, this embodiment employs the adsorption rotor **(53)** rotatable around a point between the first passage **(P1)** and the second passage **(P2)** and the heater **(58)** is disposed in the second passage (P2) upstream of the adsorption rotor **(53).** Therefore, an operation can be continuously carried out to dehumidify the first air in the side of the adsorption rotor **(53)** located across the first passage **(P1)** and concurrently regenerate the side of the adsorption rotor **(53)** located across the second passage (P2). Hence, the air supplied to the treatment space **(2)** (the first air) is always a low-humidity air and hydrogen peroxide is mixed into the low-humidity air. This enables the hydrogen peroxide concentration in the treatment space **(2)** to be always kept uniform.

It is generally known that, in sterilization systems of this kind, a higher sterilization effect can be obtained at a lower humidity in the treatment space **(2).** However, even if in conventional sterilization systems a low-humidity air is used as an air supplied into the isolator, the supply of hydrogen peroxide after the supply of low-humidity air into the space of the isolator causes humidity rise in the space, thereby deteriorating the sterilization effect. In contrast, according to this embodiment, continuous operation of the dehumidifier **(52)** provides a control to always keep the humidity of the first air constant, which prevents the deterioration of the sterilization effect.

The correlation between the air humidity and the hydrogen peroxide concentration in the treatment space **(2)** has not been clear so far. With the sterilization system **(1)** of this embodiment, there can be a way to use it to continuously measure the humidity and hydrogen peroxide concentration in the treatment space **(2)** and store the measurement results as data thereby finding an effective hydrogen peroxide concentration relative to the air humidity.

Furthermore, since the two coolers **(55, 57)** are disposed in the first passage **(P1),** one upstream of the adsorption rotor **(53)** and the other downstream of the adsorption rotor **(53)** between the adsorption rotor **(53)** and the temperature/humidity sensor **(62),** the first air is cooled by the coolers **(55, 57)** and dehumidified by the adsorption rotor **(53),** followed by mixture with hydrogen peroxide and supply to the treatment space **(2).** Particularly, since the first air is cooled upstream of the adsorption rotor **(53),** this enhances the adsorption capacity of the adsorption rotor **(53)** and supplies a lower-humidity air to the treatment space **(2).**

Furthermore, since the elements of the dehumidifier **(52)** are each made of material corrosion-resistant to hydrogen peroxide, they are not corroded by hydrogen peroxide even if hydrogen peroxide flows through them. Therefore, the dehumidifier (52) is not immediately damaged, which enables to design a good working sterilization system.

According to this embodiment, the following effects can be exhibited in addition to the effects described above.

First, since the installation **(3)** such as a drug production line **(3)** installed in the treatment space **(2)** is covered with the gas-permeable cover member **(30)** and hydrogen peroxide is introduced through the first introduction passage **(23)** to the interior of the cover member **(30),** this enhances the sterilization effect in the surroundings of the installation **(3)** and prevents the system structure from being complicated as a whole. Particularly, since the first introduction passage **(23)** is connected to the lower part of the cover member **(30),** this enhances the sterilization effect in the surroundings of the foot of the installation **(3)** likely to be dead spots and provides a desired sterilization level for a short time.

Furthermore, since in this embodiment hydrogen peroxide is supplied through the gas-permeable pores in the cover member **(30)** into the treatment space **(2)** and also supplied through the second introduction passage **(24)** into the treatment space **(2),** this provides an effect of making the concentration distribution of hydrogen peroxide in the treatment space (2) uniform.

Furthermore, since hydrogen peroxide generated in the hydrogen peroxide generator **(51)** is supplied while hydrogen peroxide in the circulating gas is decomposed by the decomposer **(41),** the hydrogen peroxide concentration in the gas can be stabilized and no hydrogen peroxide is contained in the exhaust gas.

### - Modification of Embodiment -

In the above embodiment, harmless gas may be returned into the sterilizer **(10)** by always operating the decomposer **(41)** or the gas having contained hydrogen peroxide in the treatment space **(2)** may be returned into the sterilizer **(10)** as it is by stopping the operation of the decomposer **(41).** In the latter case, when the hydrogen peroxide concentration reaches a good enough value, the hydrogen peroxide generator **(51)** is deactivated to simply circulate hydrogen peroxide.

Furthermore, in the above embodiment, the temperature and humidity of the processed air in the first passage **(P1)** is detected to operate its absolute humidity and the amount of heat applied by the heater **(58)** is controlled based on the absolute humidity to keep the humidity of the first air having passed through the adsorption rotor **(53)** constant. Alternatively, instead of the temperature/humidity sensor **(62),** the dew-point temperature sensor may be used to obtain the absolute humidity of the first air. Also in this case, the amount of heat applied by the heater **(58)** can be controlled to keep the humidity of the first air having passed through the adsorption rotor **(53)** constant.

### <<Other Embodiments>>

The above embodiment may have the following configurations.

For example, though in the above embodiment the circulation passage **(40)** is provided to circulate gas in the treatment space **(2)** therethrough, the circulation passage (40) is not necessarily provided.

Furthermore, though the above embodiment employs as a dehumidifier **(52)** a dehumidifier using an adsorption rotor **(53),** a dehumidifier of any other type capable of regenerating an adsorbent can be used instead.

Furthermore, though the above embodiment includes not only the first introduction passage **(23)** but also the second introduction passage **(24),** the second introduction passage **(24)** is not necessarily provided. The gas-permeable cover member **(30)** is not necessarily limited to non-woven fabric and may be fabricated using other gas-permeable materials.

Furthermore, the location of the installation **(3)** in the treatment space **(2)** and the relative position between the installation **(3)** and the second introduction passage **(24)** may be appropriately changed according to the shape or other factors of the treatment space **(2).** The installation **(3)** may be other than a drug production line.

So long as the present invention is configured as described so far so that in introducing hydrogen peroxide into the treatment space **(2),** hydrogen peroxide is mixed with dehumidified air and then introduced into the treatment space **(2),** the other specific configurations and structures may be appropriately changed.

The above embodiments are merely illustrative in nature and are not intended to limit the scope, applications and use of the invention.

### Industrial Applicability

As can be seen from the above, the present invention is useful for a sterilization system for subjecting a treatment space to a sterilization process with hydrogen peroxide.

## Claims

1. A sterilization system comprising a hydrogen peroxide generator **(51)** for generating hydrogen peroxide and an introduction passage **(20)** for introducing hydrogen peroxide generated in the hydrogen peroxide generator **(51)** into a treatment space **(2)** to be sterilized, wherein
said sterilization system further comprises a dehumidifier **(52)** for dehumidifying air, and
the introduction passage **(20)** comprises: an air introduction passage **(21)** for introducing the air having passed through the dehumidifier **(52)** into the treatment space **(2);** and a hydrogen peroxide introduction passage **(22)** connecting the hydrogen peroxide generator **(51)** to the air introduction passage **(21)** at a position between the dehumidifier **(52)** and the treatment space **(2).**

2. The sterilization system of claim 1, further comprising a circulation passage **(40),** connected to an exhaust port **(2a)** provided at the treatment space **(2)** and an air inlet side of the dehumidifier **(52),** for introducing gas in the treatment space **(2)** into the dehumidifier **(52).**

3. The sterilization system of claim 1, wherein the dehumidifier **(52)** comprises: an adsorption rotor **(53)** that is disposed to span a first passage **(P1)** allowing a first air to flow and a second passage (P2) allowing a second air to flow, rotatable about a point between the first passage **(P1)** and the second passage **(P2)** and capable of adsorbing moisture in the first air; and a heater **(58)** disposed in the second passage (P2) upstream of the adsorption rotor **(53).**

4. The sterilization system of claim 3, wherein the dehumidifier (52) further comprises: a temperature/humidity sensor **(62)** disposed in the first passage **(P1)** downstream of the adsorption rotor **(53);** and a regulator **(64)** for regulating the heating capacity of the heater **(58)** based on the detected value of the temperature/humidity sensor **(62).**

5. The sterilization system of claim 4, wherein the dehumidifier **(52)** further comprises coolers **(55, 57)** in the first passage **(P1),** one disposed upstream of the adsorption rotor **(53)** and one disposed downstream of the adsorption rotor **(53)** between the adsorption rotor **(53)** and the temperature/humidity sensor **(62).**

6. The sterilization system of claim 1, wherein elements of the dehumidifier **(52)** are each made of material corrosion-resistant to hydrogen peroxide.
